# EUROPÄISCHE PATENTSCHRIFT

(11) **EP 2 103 296 B1**
(45) Veröffentlichungstag und Bekanntmachung des Hinweises auf die Patenterteilung: **22.02.2012**
(21) Anmeldenummer: 08102842.5
(22) Anmeldetag: 20.03.2008
(51) Int. Cl.: A61K 6/083

(54) **Polymerbeschichteter Glasfüllstoff zur Verwendung in Dentalwerkstoffen**
Polymer-coated glass filler for use in dental material
Matière de remplissage de verre revêtue de polymère et destinée à être utilisée dans des matières dentaires

(43) Veröffentlichungstag der Anmeldung: 23.09.2009
(73) Patentinhaber: Ivoclar Vivadent AG, 9494 Schaan (LI)
(72) Erfinder: Moszner, Norbert, 9493 Mauren (LI); Angermann, Jörg, 7320, Sargans (CH); Rheinberger, Volker, 9490, Vaduz (LI); Voser, Dieter, 9494, Schaan (LI); Vogel, Karin, 9487, Gamprin (LI); Klapdohr, Simone, 83022, Rosenheim (DE)
(74) Vertreter: UEXKÜLL & STOLBERG

(56) Entgegenhaltungen:
- WO-A-2004/103319
- DE-A1- 2 139 315
- US-A- 3 826 778

## Beschreibung

Die vorliegende Erfindung betrifft mit Vinylchlorid Homo-oder Co-Polymer behandelte Glasfüllstoffe, die zur Verwendung in Dentalwerkstoffen, insbesondere zur Herstellung von selbsthaftenden Kompositen, geeignet sind.

Komposite werden in der restaurativen Zahnheilkunde als Füllungs- und Befestigungsmaterialien oder in der Kieferorthopädie als Zemente verbreitet eingesetzt. Komposite sind allgemein als Kombination zweier Stoffe definiert, deren Eigenschaften sich von denen der reinen Komponenten unterscheiden. Im Falle von dentalen Kompositen handelt es sich um Mehrstoffsysteme, die aus einer organischen Monomer- bzw. Polymermatrix bestehen, in der ein oder mehrere Füllstoffe eingebaut sind.

Dabei haben die Füllstoffe hauptsächlich die Funktion, die mechanischen Eigenschaften eines dentalen Komposits, wie z.B. die Festigkeit, Härte oder den Elastizitätsmodul, zu verbessern, die thermische Ausdehnung und den Polymerisationsschrumpf zu verringern und die rheologischen und optischen Eigenschaften der Komposite gezielt zu beeinflussen. Die dafür eingesetzten dentalen Füllstoffe lassen sich in rein organische und anorganische Füllstoffe bzw. Kombinationen daraus einteilen, wobei am häufigsten anorganische Füllstoffe zum Einsatz kommen. Diese lassen sich wiederum in oxidische und nichtoxidische Füllstoffe unterteilen. Die oxidischen Füllstoffe klassifiziert man dann nochmals in silicatische und nichtsilicatische Füllstoffe.

Zu den silicatischen Füllstoffen gehören gemahlene Gläser, wie z.B. Barium-Silicat-Gläser, Strontium-Silicat-Gläser, Lithium-Aluminium-Silicat-Gläser und röntgenopaque Aluminium-Fluoro-Silicat-Gläser, die vor allem in methacrylatharzverstärkten Glasionomerzementen Verwendung finden. Zu den silicatischen Füllstoffen zählen auch reine Siliciumdioxid-Füllstoffe, die ebenfalls häufig in Dentalmaterialien Anwendung finden. Bekannt sind auch Mischoxide auf Basis von Silicium- und Zirconoxid beziehungsweise Kern-Schale-Systeme. Diese werden neben der verstärkenden Wirkung zur Erhöhung der Röntgenopazität und zur Einstellung der Transparenz, durch Anpassung des Brechungsindex in Abhängigkeit von der Füllerzusammensetzung, eingesetzt.

Nichtsilicatische Füllstoffe wie z.B. Zirconoxid, Tantaloxid, Ytterbiumtrifluorid oder Yttriumoxid werden als Röntgenkontrastmittel verwendet. Aluminium- und Titanoxid dienen wegen ihres hohen Brechungsindex häufig als Trübungsmittel.

Dentale Füllstoffe sollten in der Regel farblos, im Mundmilieu beständig und toxikologisch unbedenklich sein. Zur Verbesserung der mechanischen Eigenschaften werden die Oberflächen der dentalen Füllstoffe, die z.B. in den verbreitet eingesetzten lichthärtenden Kompositen enthalten sind, mit polymerisationsfähigen Silanen, wie z.B. (Meth)acryloyloxyalkyltrialkoxysilanen, funktionalisiert. Die eingeführten (Meth)acrylatgruppen werden dann bei der Aushärtung des Komposits durch Copolymerisation kovalent mit der Polymermatrix verbunden.

Gegenwärtig finden Füllungskomposite mit selbsthaftenden Eigenschaften zunehmendes Interesse (N. Moszner, U. Salz, Macromol. Mater. Eng. 292 (2007) 245-271). Dabei handelt es sich um Komposite, die neben den herkömmlichen Vernetzer- bzw. Verdünnermonomeren, wie z.B. Bis-GMA (2,2-Bis[4-(2-hydroxy-3-methacryloyloxypropoxy)phenyl]propan) oder UDMA (1,6-Bis[2-methacryloyl-oxyethoxycarbonylamino]-2,4,4-trimethylhexan) bzw. D₃MA (Decandioldimethacrylat) oder TEGDMA (Triethylenglykoldimethacrylat), stark acide Säuremonomere enthalten. Solche Säuremonomere, wie z.B. auch GDMP (Glycerindimethacrylatdihydrogenphosphat) oder MDP (10-Methacryloyloxydecyl-dihydrogenphosphat) sind in der Lage, die Haftung auf Dentin und Zahnschmelz zu vermitteln.

Werden für selbsthaftende Komposite die herkömmlichen Glasfüllstoffe, wie z.B. Barium-Silicat-Gläser, eingesetzt, so kann es zu nachteiligen Effekten kommen. Zum einen können die sauren Monomere der organischen Matrix aus den Partikeln der Glasfüllstoffe Kationen, z.B. Ba²⁺-Ionen, herauslösen. Im Ergebnis dessen kann es zur Salzbildung und damit verbunden zu einer deutlichen Viskositätserhöhung der organischen Matrix bzw. zur spürbaren Verdickung des Komposits und damit zu einer Verschlechterung der Lagestabilität kommen. Ferner besteht die Gefahr, dass die sauren Monomermoleküle durch die Reaktion der aciden Phosphatgruppen mit dem Füllstoff an die Füllstoffoberfläche gebunden werden und damit nicht mehr als Haftvermittler für die Zahnhartsubstanz zur Verfügung stehen.

Nach dem Stand der Technik ist die Beschichtung von dentalen Glasfüllstoffen schon relativ lange bekannt. So beschreiben z.B. EP 0 047 971 B1 oder CH 652 139 A5 die Beschichtung von dentalen Füllstoffteilchen mit einem kalt-, licht- oder hitzehärtbaren Kunststoff, z.B. auf Dimethacrylatbasis.

Zahlreiche Veröffentlichungen existieren zur Beschichtung von dentalen Glaspulvern mit Homo- oder Heteropolysiloxanen oder Sol-Gel-Produkten, d.h. hydrolytischen Kondensaten von verschiedenen Silanen und Metallalkoxiden, wie Zr-, Ti- oder Al-Alkoxiden bzw. deren Mischungen (vgl. S. Klapdohr, N. Moszner, Monatsh. Chem. 136 (2005) 21-45).

In der US 6,620,861 B1 werden z.B. dentale Füllstoffe beschrieben, bei denen Glaspulverpartikel mit Polysiloxanen beschichtet sind.

Die Beschichtung der Glasfüllstoffe in den vorgenannten Dokumenten erfolgt aus unterschiedlichen Gründen und soll zu vorteilhaften Eigenschaften der diese Glasfüllstoffe enthaltenden Dentalwerkstoffe führen. Jedoch ist in obigen Dokumenten weder die Verwendung der Glasfüllstoffe in selbsthaftenden Kompositen beschrieben, noch ist deren mögliche Eignung zur Verwendung in selbsthaftenden Kompositen Gegenstand der offenbarten Lehren.

Auch die US 5,453,456 betrifft keine selbsthaftenden Komposite, sondern Glasionomerzemente und darin enthaltene Aluminium-Fluoro-Silicat-Glasfüllstoffe. Zur Verbesserung der mechanischen Eigenschaften des gehärteten Zements und unter Beibehaltung oder Steigerung der erwünschten Freisetzung von Fluoridionen werden die Aluminium-Fluoro-Silicat-Glasfüllstoffpartikel mit einer ionische Carboxylgruppen und Siloxygruppen aufweisenden Beschichtung versehen. Zur weiteren Verbesserung der Festigkeit und Bruchzähigkeit können die so behandelten Aluminium-Fluoro-Silicat-Glasfüllstoffe optional mit einer zusätzlichen organischen Verbindung behandelt werden. In Frage kommende zusätzliche organische Verbindungen umfassen eine umfangreiche Liste monomerer, oligomerer und polymerer Verbindungen, wobei als Polymere unspezifisch Polymere verschiedener Arten genannt werden, z.B. Polykondensate, Polyaddukte und Polymerisate, darunter unter anderem Polyvinylchlorid. Bevorzugte zusätzliche organische Verbindungen enthalten ethylenisch ungesättigte Gruppen und hydrophile Gruppen, wie etwa Ethylenglykolgruppen, und in allen Ausführungsbeispielen, in denen eine Behandlung mit einer zusätzlichen organischen Verbindung durchgeführt wird, werden eben Verbindungen dieser bevorzugten Art verwendet. Eine Beschichtung der Aluminium-Fluoro-Silicat-Glasfüllstoffpartikel mit Polyvinylchlorid ist nicht beschrieben.

Das Dokument US 3826778 beschreibt einen Füllstoff, der Glaspartikel enthält, die in einer Polymermatrix eingebettet sind, die aus Polyvinylchlorid besteht. Bei dem Glas handelt es sich um ein Silicatglas, das zwischen 15 und 35 Gew.% Bariumoxid enthältund röntgenopak ist.

Das Dokument DE 2139315 offenbart ein Verfahren zur Beschichtung von anorganischen Fasern, wobei die anorganischen Fasern mit einer Dispersion eines filmbildenden Polymers in einer organischen Flüssigkeit behandelt werden. Die Fasern sind E-Glas, das bekanntlich im alkalischem Medium angegriffen wird. Als geeignetes Polymer wird Polyvinylchlorid zitiert. Als Lösungsmittel wird THF verwendet. Das Glas kann silanisiert sein.

Der vorliegenden Erfindung liegt die Aufgabe zugrunde, Glasfüllstoffe bereitzustellen, die bei Kombination mit sauren organischen Matrixmaterialien zu einer im Vergleich mit konventionellen Glasfüllstoffen deutlich erhöhten Stabilität der Pastenviskosität (Konsistenz) führen, ohne die Aushärtungsgeschwindigkeit der Komposite oder die mechanischen Eigenschaften der gehärteten Komposite nachteilig zu beeinflussen. Ziel der Erfindung ist insbesondere die Bereitstellung von selbsthaftenden Kompositen, als Dentalwerkstoffe deren Verarbeitungseigenschaften durch Lagerung nicht beeinträchtigt wird.

Die Aufgabe wird erfindungsgemäß gelöst durch einen Füllstoff auf der Basis von Glaspartikeln, der dadurch gekennzeichnet ist, dass die Glaspartikel auf der Oberfläche ein Homo- oder Copolymer von Vinylchlorid aufweisen.

Die vorliegende Erfindung betrifft ferner einen Dentalwerkstoff, der diesen Füllstoff enthält, ebenso die Verwendung des Füllstoffs zur Herstellung eines Dentalwerkstoffs.

Gegenstand der vorliegenden Erfindung ist außerdem ein Verfahren zur Herstellung eines Füllstoffs auf der Basis von Glaspartikeln, bei dem man Glaspartikel in einer Lösung eines Homo- oder Copolymers von Vinylchlorid in einem organischen Lösungsmittel dispergiert und das Lösungsmittel anschließend entfernt.

Die im Folgenden verwendeten Begriffe "Polyvinylchlorid(e)" bzw. "PVC" umfassen sowohl Homo- als auch Copolymere von Vinylchlorid.

Die Glaspartikel, die die Basis des erfindungsgemäßen Füllstoffs bilden, können grundsätzlich aus allen Gläsern bestehen, die im Dentalbereich üblicherweise als Füllstoffe eingesetzt werden, in der Regel Silicatgläser.

Vorzugsweise bestehen die Glaspartikel aus einem Glas, das Bariumoxid und/oder Strontiumoxid enthält. Besonders bevorzugt sind Gläser die 15 bis 35 Gew.-% Bariumoxid und/oder 15 bis 25 Gew.-% Strontiumoxid enthalten.

Weiter bevorzugt sind Gläser, die in mikronisierter Form bei Dispersion in Wasser eine alkalische Reaktion zeigen. Hierunter fallen vorzugsweise Gläser, die, wenn 2 g Glaspartikel mit einer gewichtsmittleren Partikelgröße von etwa 1,5 µm in 50 ml destilliertem Wasser (pH = 7) durch Rühren bei ca. 20 °C suspendiert werden, nach 30 min eine Erhöhung des pH-Werts des Wassers auf mindestens 8, besonders bevorzugt mindestens 8,5 bewirken.

In einer bevorzugten Ausführungsform ist das verwendete Glas fluoridarm oder fluoridfrei. Hierunter werden Gläser mit einem Fluoridgehalt von 0 bis 15 Gew.-%, bevorzugt 0 bis 10 Gew.-%, besonders bevorzugt 0 bis 3 Ges.-% und ganz besonders bevorzugt von < 1 Gew.-% verstanden.

Bei Dentalwerkstoffen wird häufig eine ausreichende Radioopazität gefordert, um zu ermöglichen, dass der Werkstoff auf einer Röntgenaufnahme vom natürlichen Zahn unterscheidbar ist. Ist der erfindungsgemäße Füllstoff zur Verwendung in radioopaken Dentalwerkstoffen vorgesehen, ist es daher vorteilhaft, ein radioopakes Glas einzusetzen. Im Allgemeinen zeigen Gläser, die schwere Metallionen (Massenzahl > 37) enthalten, eine ausreichende Radioopazität. Beispiele für bekannte radioopake Gläser, die bei dem Füllstoff der vorliegenden Erfindung vorzugsweise eingesetzt werden, sind Barium-Silicat-Glas (insbesondere Barium-Aluminium-Silicat-Glas) und Strontium-Silicat-Glas (insbesondere Strontium-Aluminium-Silicat-Glas). Bevorzugte Zusammensetzungen dieser Gläser sind 40 bis 60 Ges.-% SiO₂, 10 bis 15 Ges.-% Al₂O₃, 10 bis 20 Ges.-% B₂O₃ und 15 bis 35 Gew.-% BaO (Barium-Aluminium-Silicat-Glas) oder 40 bis 60 Gew.-% SiO₂, 10 bis 15 Gew.-% Al₂O₃, 10 bis 20 Gew.-% B₂0₃, 0 bis 3 Gew.-% BaO und 15 bis 25 Gew.-% SrO (Strontium-Aluminium-Silicat-Glas).

Die als Ausgangsmaterial verwendeten Glaspartikel weisen vorzugsweise eine mittlere Partikelgröße im Bereich von 0,01 bis 10 µm, besonders bevorzugt von 0,5 bis 5 µm und ganz besonders bevorzugt 0,6 bis 2,0 µm auf. Bei der mittleren Partikelgröße handelt es sich um das Gewichtsmittel, bestimmt durch Lichtstreuung. Es ist ferner vorteilhaft, wenn die Glaspartikel keine Partikel, die größer als 90 µm, bevorzugt größer als 20 µm sind, enthalten, d.h. die Glaspartikel ein Sieb mit den vorgenannten Maschenweiten passieren können.

Die Glaspartikel weisen vorzugsweise eine spezifische Oberfläche - bestimmt mit der BET-Methode nach ISO 9277:1995 - von 1 bis 30 m²/g, vorzugsweise 1 bis 20 m²/g und besonders bevorzugt vom 3 bis 10 m²/g auf.

Glaspartikel geeigneter Größen lassen sich beispielsweise auf übliche Weise durch Vermahlen herstellen.

Zur Verbesserung der mechanischen Eigenschaften des ausgehärteten Dentalwerkstoffs ist es vorteilhaft, einen erfindungsgemäßen Füllstoff auf Basis eines silanisierten Glases zu verwenden. Unter "Silanisierung" versteht man die Funktionalisierung der Glasoberfläche mit polymerisationsfähigen Silanen, etwa durch Umsetzung mit (meth)acrylatfunktionalisierten Silanen, z.B. (Meth)acryloyloxyalkyltrialkoxysilanen, üblicherweise 3-(Methacryloyloxy)propyltrimethoxysilan, 3-(Methacryloyloxy)propyltriethoxysilan, 3-(Methacryloyloxy)propyltrichlorsilan, Methacryloyloxymethyltrimethoxysilan, Methacryloyloxymethyltriethoxysilan, 3-(Methacryloyloxy)propylmethyldichlorsilan oder 3-(Methacryloyloxy)propylmethyldimethoxysilan. 3-(Methacryloyloxy)propyltrimethoxysilan ist bevorzugt. Die Silanisierung der Gläser erfolgt auf herkömmliche Weise und ist dem Fachmann bekannt.

Die Glaspartikel des erfindungsgemäßen Füllstoffs weisen auf der Oberfläche ein Homo- oder Copolymer von Vinylchlorid auf. Üblicherweise ist die Oberfläche der Glaspartikel mindestens teilweise, vorzugsweise größtenteils oder vollständig mit dem Polyvinylchlorid beschichtet.

Für die erfindungsgemäßen Füllstoffe geeignete Polyvinylchloride sind beispielsweise handelsübliche Homo- oder Copolymere von Vinylchlorid, vorzugsweise mit zahlenmittleren Molekulargewichten Mₙ im Bereich von 30.000 bis 130.000, vorzugsweise 30.000 bis 50.000. Neben den insbesondere geeigneten Homopolymeren von Vinylchlorid, vorzugsweise mit den oben genannten Molekulargewichten, können auch Copolymere von Vinylchlorid eingesetzt werden. Diese enthalten in einer bevorzugten Ausführungsform mindestens 80 mol-% polymerisierte Vinylchlorideinheiten und weniger als 20 mol-% polymerisierte Einheiten eines oder mehrerer Comonomere. Geeignete Comonomere sind z.B. Vinylacetat, Acrylnitril, Ethylen und Vinylidenchlorid, wobei Ethylen und/oder Acrylnitril bevorzugt sind.

Für die vorliegende Erfindung ist es nicht entscheidend, nach welchem Verfahren das PVC hergestellt wurde. Übliche Herstellungsverfahren umfassen Suspensionspolymerisation, Emulsionspolymerisation und Massepolymerisation, wobei Suspensionspolymerisation technisch die größte Bedeutung hat.

Zur Aufbringung des PVC auf die Glaspartikel, die wie oben beschrieben silanisiert sein können, wird das PVC in einem organischen Lösungsmittel gelöst und die Glaspartikel in dieser Lösung dispergiert.

Als Lösungsmittel geeignet sind hierbei alle organischen Lösungsmittel, in denen PVC in einer ausreichenden Konzentration löslich ist. Gut geeignet sind beispielsweise cyclische Ketone, etwa Cyclopentanon und Cyclohexanon; Dimethylformamid; Dimethylsulfoxid und insbesondere Tetrahydrofuran (THF) sowie Mischungen dieser Lösungsmittel. Vorzugsweise beträgt der Massengehalt von PVC in der Lösung 0,01 bis 20 Gew.-%, besonders bevorzugt 1 bis 7 Gew.-%.

Die Glaspartikel werden in der PVC-Lösung dispergiert, vorzugsweise unter Rühren, dann nach ausreichender Zeit, üblicherweise nach einem Zeitraum von 0,2 bis 2 h, von der Lösung wieder abgetrennt, etwa durch Zentrifugation oder Filtration, und anschließend getrocknet, z.B. im Feinvakuum (10² bis 10⁻¹ Pa). Dabei ist beim Trocknen darauf zu achten, dass die Temperatur mindestens ca. 30 °C unterhalb der Glasübergangstemperatur (T_{G}) des PVC liegt. So sollte im Falle von homopolymerem PVC (T_{G} = 81 °C) die Temperatur 50 °C nicht übersteigen, damit ein Verkleben der Füllstoffpartikel infolge einer erweichten Polymerbeschichtung verhindert wird.

Die auf die Glaspartikel aufgebrachte Menge an PVC kann beispielsweise durch Röntgenfluoreszenzanalyse (XRF) erfolgen. Die PVC-Menge läßt sich aus der durch XRF ermittelten Chlormenge berechnen. Typischerweise wird das PVC in einer solchen Menge auf die Glaspartikel aufgebracht, dass das Gewicht des PVC 0,1 1 bis 7,0 Ges.-%, bevorzugt 1,0 bis 5,0 Gew.-% des Gesamtgewichts der Füllstoffpartikel ausmacht.

Die erfindungsgemäßen Füllstoffe werden vorzugsweise zur Herstellung von Dentalwerkstoffen, insbesondere selbsthaftenden Kompositen, verwendet. Die Komposite eignen sich besonders als dentale Zahnfüllungsmaterialien. Grundsätzlich können die erfindungsgemäßen Füllstoffe jedoch auch in anderen Anwendungen eingesetzt werden, etwa solchen, bei denen der Schutz der Glaspartikel vor einem umgebenden sauren Milieu von Interesse ist.

Gegenstand der vorliegenden Erfindung ist auch ein Dentalwerkstoff, üblicherweise in pastöser Form, der den erfindungsgemäßen Füllstoff wie oben einschließlich seiner bevorzugten Ausführungsformen beschrieben enthält. Vorzugsweise ist der Dentalwerkstoff ein selbsthaftendes Komposit.

Gemäß einer bevorzugten Ausführungsform enthält der Dentalwerkstoff:
(a) 1 bis 85 Gew.-%, insbesondere 5,0 bis 50 Gew.-% erfindungsgemäßen Füllstoff wie oben, einschließlich seiner bevorzugten Ausführungsformen, beschrieben,
(b) 0,1 bis 5,0 Ges.-%, insbesondere 0,2 bis 2,0 Ges.-% Initiator für die radikalische Polymerisation und
(c) 1 bis 70 Ges.-%, insbesondere 5,0 bis 40 Gew.-% eines oder mehrerer säuregruppenfreier radikalisch polymerisierbarer Monomere.

Dentalwerkstoffe zur Verwendung als selbsthaftende Zemente oder Komposite enthalten darüber hinaus zusätzlich:
(d) 1 bis 30 Gew.-%, insbesondere 3,0 bis 20 Ges.-% eines oder mehrerer säuregruppenhaltiger radikalisch polymerisierbarer Monomere.

Vorzugsweise enthält der Dentalwerkstoff kein zugesetztes Lösungsmittel.

Alle Angaben beziehen sich jeweils auf das Gesamtgewicht des Dentalwerkstoffs.

Komponente (b), der Initiator für die radikalische Polymerisation, wird ausgewählt, je nachdem, ob für den erfindungsgemäßen Dentalwerkstoff Strahlungshärtung (photochemische radikalitsche Polymerisation), Heißhärtung oder Härtung bei Raumtemperatur erwünscht ist. Unter dem Begriff "Initiator" sind auch Initiatorsysteme aus verschiedenen Verbindungen zu verstehen.

Beispiele für geeignete Photoinitiatoren umfassen Benzophenon, Benzoin sowie deren Derivate und α-Diketone und deren Derivate, wie 9,10-Phenanthrenchinon, 1-Phenyl-propan-1,2-dion, Diacetyl und 4,4'-Dichlorbenzil. Bevorzugt werden Campherchinon, 2,2-Methoxy-2-phenyl-acetophenon oder α-Diketone jeweils in Kombination mit Aminen als Reduktionsmittel, wie z.B. 4-(Dimethylamino)-benzoesäureester, N,N-Dimethylaminoethylmethacrylat, N,N-Dimethyl-sym.-xylidin oder Triethanolamin, sowie Monobenzoyl- oder Dibenzoylgermanium-Derivate verwendet.

Als Initiatoren für die Heißhärtung eignen sich beispielsweise Benzpinakol und 2,2'-Dialkylbenzpinakole.

Als Initiatoren für eine bei Raumtemperatur durchgeführte Polymerisation (Kalthärtung) können beispielsweise Redox-Initiatorkombinationen, wie z.B. Kombinationen von Benzoylperoxid mit N,N-Dimethyl-sym.-xylidin oder N,N-Dimethyl-p-toluidin, verwendet werden. Darüber hinaus sind auch Redoxsysteme, bestehend aus Peroxiden und Reduktionsmitteln, wie z.B. Ascorbinsäure, Barbituraten oder Sulfinsäuren, geeignet.

Komponente (c), die säuregruppenfreien radikalisch polymerisierbaren Monomere, und Komponente (d), die säuregruppenhaltigen radikalisch polymerisierbaren Monomere, machen gemeinsam das Bindemittel des erfindungsgemäßen Dentalwerkstoffs aus, das beim Aushärten radikalisch polymerisiert.

Die Bindemittelmonomere der Komponente (c) umfassen sogenannte Vernetzermonomer(e) mit mindestens zwei, vorzugsweise 2 bis 4 radikalisch polymerisierbaren Gruppen und sogenannte Verdünnermonomer(e), die sich aufgrund ihrer Viskosität und guten Löslichkeit zum Verdünnen von Polymerisationsharzen eignen. Verdünnermonomere können eine oder auch mehrere polymerisierbare Gruppen enthalten und im letzteren Fall auch als Vernetzermonomere wirken.

Als Vernetzermonomere eignen sich vor allem vernetzende bi-oder mehrfunktionelle Methacrylate und Acrylate, wie z.B. 1,6-Bis[2-methacryloyl-oxyethoxycarbonylamino]-2,4,4-trimethylhexan (UDMA), 2,2-Bis[4-(2-hydroxy-3-methacryloyloxypropoxy)phenyl]propan (Bis-GMA), 1,6-Bis[2-acryloyl-oxyethoxycarbonylamino]-2,4,4-trimethylhexan, Diethylenglykoldimethacrylat, Triethylenglykoldimethacrylat (TEGDMA), Diethylenglykoldiacrylat, Triethylenglykoldiacrylat, Trimethylolpropantrimethacrylat, Trimethylolpropantriacrylat, Pentaerythrittetramethacrylat, Pentaerythrittetraacrylat sowie Butandioldimethacrylat, Butandioldiacrylat, 1,10-Decandioldimethacrylat (D;MA), 1,10-Decandioldiacrylat, 1,12-Dodecandioldimethacrylat und 1,12-Dodecandioldiacrylat, die durch Veresterung von (Meth)acrylsäure mit den entsprechenden Di- oder Polyolen zugänglich sind. Darüber hinaus sind auch hydrolysestabile Vernetzermonomere geeignet, beispielsweise Urethane aus 2-(Hydroxymethyl)acrylsäure und Diisocyanten, wie 2,2,4-Trimethylhexamethylendiisocyanat und Isophorondiisocyanat; vernetzende Pyrrolidone, wie z.B. 1,6-Bis(3-vinyl-2-pyrrolidonyl)-hexan; kommerziell zugängliche Bisacrylamide bzw. Bis(meth)acrylamide, wie Methylen- und Ethylenbisacrylamid, N,N'-Diethyl-1,3-bis(acrylamido)-propan, 1,3-Bis(methacrylamido)-propan, 1,4-Bis(acrylamido)-butan und 1,4-Bis(acryloyl)-piperazin, die durch Umsetzung aus den entsprechenden Diaminen mit (Meth)acrylsäurechlorid synthetisiert werden können.

Als "hydrolysestabil" werden hierin solche Monomere bezeichnet, die in Wasser oder in Mischungen von Wasser und wassermischbaren Lösungsmitteln bei einer Konzentration von ca. 20 Ges.-% und einem pH-Wert von ca. 2,0 bei 37°C für mindestens 6 Wochen stabil sind, d.h. zu weniger als 5 % hydrolysieren.

Als Verdünnermonomere werden hierin bevorzugt flüssige Monomere mit einer viskosität η von kleiner als 100 mPa·s, gemessen bei 20°C, verwendet. Beispiele für Verdünnermonomere umfassen hydrolysestabile Mono(meth)acrylate, z.B. Mesitylmethacrylat; 2-(Alkoxymethyl)acrylsäuren, z.B. 2-(Ethoxymethyl)acrylsäure, 2-(Hydroxymethyl)acrylsäure; N-mono- oder -disubstitiuierte Acrylamide, wie z.B. N-Ethylacrylamid, N,N-Dimethylacrylamid, N-(2-Hydroxyethyl)acrylamid und N-Methyl-N-(2-hydroxyethyl)-acrylamid bzw. N-monosubstituierte Methacrylamide, wie z.B. N-Ethylmethacrylamid und N-(2-Hydroxyethyl)methacrylamid. Außerdem lassen sich auch N-Vinylpyrrolidon, 2-(Methacryloyloxy)-ethylacetoacetat oder Allylether als Verdünnermonomere einsetzen.

Komponente (d), die säuregruppenhaltigen radikalisch polymerisierbaren Monomere, ist für eine verbesserte Haftung des Dentalwerkstoffs an Schmelz/Dentin verantwortlich. Es handelt sich hierbei um stark saure Monomere, auch Adhäsivmonomere genannt, die zum einen bei der Anwendung des Dentalwerkstoffs eine leichte Schmierschicht ("Smear-Layer") auf dem Schmelz/Dentin entfernen und zum anderen den Schmelz bzw. das Dentin anätzen, so dass Monomere eindiffundieren können und bei der anschließenden Polymerisation unter Bildung von sogenannten Polymer-Tags zu einer starken Bindung zwischen dem gehärteten Dentalwerkstoff und Schmelz/Dentin führen.

Die Adhäsivmonomere (d) enthalten vorzugsweise 1 bis 4 Säuregruppen. Bevorzugte Säuregruppen sind Carbonsäure-, Sulfonsäure-, Phosphonsäure- und/oder Phosphorsäuregruppen. Verbindungen, die als Säuregruppen Carbonsäure-, Phosphonsäure-und/oder Phosphorsäuregruppen enthalten, sind besonders bevorzugt. Verbindungen mit mehr als einer Säuregruppen können unterschiedliche Säuregruppen oder vorzugsweise identische Säuregruppen enthalten. Besonders vorteilhafte Adhäsivmonomere sind polymerisationsfähige Dihydrogen- und Hydrogenphosphate.

Als Adhäsivmonomere (d) geeignete Beispiele umfassen Glycerindimethacrylatdihydrogenphosphat (GDMP), 4-(Meth)acryloyloxyethyl-trimellitsäureanhydrid, 10-Methacryloyloxydecylmalonsäure, N-(2-Hydroxy-3-meth-acryloyloxypropyl)-N-phenylglycin, 4-Vinylbenzoesäure, 2-Methacryloyloxyethyl-phenyl-hydrogenphosphat, 10-Methacryloyloxydecyl-dihydrogenphosphat (MDP), 2-Methacryloyloxyethyl-dihydrogenphosphat und Dipentaerythritpentamethacryloyloxyphosphat. Vorteilhaft sind auch hydrolysestabile Adhäsivmonomere, wie 4-Vinylbenzylphosphonsäure, 2-[4-(Dihydroxyphosphoryl)-2-oxa-butyl]-acrylsäure sowie deren Amide und hydrolysestabile Ester, wie z.B. 2-[4-(Dihydroxyphosphoryl)-2-oxa-butyl]-acrylsäure-2,4,6-trimethylphenylester, sowie (Meth)acrylamiddihydrogenphosphate, wie z.B. 6-Methacrylamidohexyl- und 1,3-Bis(methacrylamido)-propan-2-yl-dihydrogen-phosphat.

Alle oben für die einzelnen Komponenten beispielhaft genannten Monomere können jeweils allein oder in Mischungen eingesetzt werden.

Die erfindungsgemäßen Dentalwerkstoffe enthalten vorzugsweise keine Polymere oder Copolymere mit Säuregruppen.

Die erfindungsgemäßen Dentalwerkstoffe können die erfindungsgemäßen Füllstoffe auch in Mischung mit einem oder mehreren anderen üblichen Füllstoffen (e), wie z.B. anorganischen, kugelförmigen, amorphen Füllstoffen auf der Basis von Oxiden, wie ZrO₂, Ta₂O₃ und TiO₂ bzw. Mischoxiden aus SiO₂, ZrO₂ und/oder TiO₂, nanopartikulären oder mikrofeinen Füllstoffen, wie pyrogener Kieselsäure, Fällungskieselsäure oder Ytterbiumtrifluorid, sowie sogenannten "Isofüllern", d.h. Pulvern, die durch Mahlen von gehärteten Kompositen zugänglich sind, enthalten. Bevorzugte gewichtsmittlere Partikelgrößen der oben genannten Füllstoffe liegen für die oxidischen Füllstoffe im Bereich von 10 bis 200 nm, für die nanopartikulären und mikrofeinen Füllstoffe im Bereich von 20 nm bis 5 µm und für die Isofüller im Bereich von 5 bis 100 µm.

Gegebenenfalls können die erfindungsgemäßen Dentalwerkstoffe weitere Additive (f) enthalten, z.B. Stabilisatoren, Aromastoffe, Farbmittel, mikrobiocide Wirkstoffe, fluoridionenabgebende Additive, optische Aufheller, Weichmacher und/oder UV-Absorber.

Zur Herstellung der erfindungsgemäßen Dentalwerkstoffe werden der erfindungsgemäße Füllstoff (a) und die weiteren oben beschriebenen Komponenten (b), (c) und gegebenenfalls (d) und weiterer Füllstoff (e) und Additiv(e) (f) vermischt.

Es wurde festgestellt, dass das PVC auf der Oberfläche der Glaspartikel des erfindungsgemäßen Füllstoffs bei Verwendung desselben in einer sauren organischen Matrix, etwa in einem selbsthaftenden Komposit, das Herauslösen von Kationen, z.B. Ba²⁺-Ionen, durch die Säurebestanteile der Matrix deutlich erschwert, damit eine Salzbildung und Viskositätserhöhung der organischen Matrix vermindert wird und schließlich eine signifikante Verbesserung der Lagerstabilität der pastösen Dentalwerkstoffe erreicht werden kann. Dies ist insofern erstaunlich, als gemäß dem Stand der Technik die Freisetzung beispielsweise von Fluoridionen durch die Oberflächenbehandlung von Füllstoffen mit polymeren organischen Verbindungen nicht behindert oder sogar verbessert wird.

PVC ist in den üblichen Monomeren der Matrix, insbesondere den Bindemittelmonomeren, die zuvor als Komponente (c) genannt wurden, wie z.B. Bis-GMA, UDMA, TEGDMA oder D₃MA, sowie den stark sauren Adhäsivmonomeren der Komponente (d), wie z.B. MDP oder GDMP, praktisch unlöslich. Deshalb kommt es bei der Herstellung der Dentalwerkstoffe mit einer Monomermischung, die z.B. auf den vorgenannten Monomeren basiert, nicht zu einer Ablösung der PVC-Beschichtung. Darüber hinaus hat sich überraschend gezeigt, dass die mechanischen Eigenschaften, z.B. die Biaxialfestigkeit, des gehärteten Dentalwerkstoffs durch die PVC-Beschichtung nicht nachteilig beeinflusst werden. Es wird vermutet, dass die relativ große Polarität von PVC nicht nur vorteilhaft für die Filmbildung auf der Glaspartikeloberfläche ist, sondern anscheinend auch für den Füllstoff-Matrix-Verbund im Komposit. Die PVC-Beschichtung verringert die Aushärtungsgeschwindigkeit der Dentalwerkstoffe nicht, und die Dentinhaftung der Dentalwerkstoffe wird nicht beeinträchtigt.

Die Erfindung wird im Folgenden anhand von Beispielen näher erläutert.

### Bezugsbeispiel 1: Silanisierung eines Glasfüllers

93 g Glaspartikel bestehend aus einem Barium-Aluminium-Silicat-Glas mit gewichtsmittlerer Korngröße d₅₀ von 1,5 µm (Fa. Schott AG, Mainz; Zusammensetzung: 55 Gew.-% SiO₂, 25 Ges.-% BaO, 10 Gew.-% B₂O₃ und 10 Ges.-%. Al₂O₃) wurden unter Zugabe von 5 g 3-(Methacryloyloxy)-propyltrimethoxysilan und 2 g Wasser silanisiert. Dabei wurde der Glasfüller im Turbularmischer (Planetenmischer) vorgelegt, dann das Wasser zugegeben und vermischt. Danach wurde das Silan zugegeben und über Nacht gemischt. Anschließend wurde der Glasfüller durch ein Sieb (Maschengröße 200 µm) gesiebt und 48 h bei 50°C getrocknet. Der Glasfüller wurde zuletzt durch ein Sieb mit der Maschengröße 90 µm gesiebt. Nach der Silanisierung betrug der organische Anteil, der dem Glühverlust entspricht, 2,8 Ges.-%. Für die spezifische Oberfläche des Glasfüllers wurde nach der üblichen BET-Methode (ISO 9277:1995) ein Wert von 3,9 m²/g bestimmt. Dabei erfolgte die Messung der spezifischen Oberfläche mittels N₂-Sorption mit einem Gerät NOVA 2200e der Fa. Quantachrome mit 6 Messpunkten nach Proben-Entgasung über 18 h bei 105°C im Vakuum (< 10 mbar). Im Glasfüller konnte mittels Röntgenfluoreszenzanalyse (XRF) kein Chlor nachgewiesen werden.

### Beispiel 2: Beschichtung des silanisierten Glasfüllers mit Polyvinylchlorid

60 g des silanisierten Glasfüllers aus Bezugsbeispiel 1 wurden in 300 g einer 5,0-gew.-%igen Lösung von Polyvinylchlorid (Fa. Aldrich; Mₙ ca. 35.000) in THF 30 min dispergiert und anschließend abfiltriert. Das Pulver wurde 5 h am Rotationsverdampfer bei 40°C getrocknet, Agglomerate wurden aufgeteilt und es wurde über ein Sieb mit der Maschengröße 90 µm gesiebt. Der organische Anteil, der über Glühverlust bestimmt wurde, beträgt 4,9 Ges.-%. Die spezifische Oberfläche des Glasfüllers nach BET (wie in Bezugsbeispiel 1 beschrieben bestimmt) betrug 4,0 m²/g. Die XRF ergab einen Cl-Gehalt von 1,3 %, was einem PVC-Gehalt von 2,3 % entspricht

### Vergleichsbeispiel 3 und Beispiel 4: Herstellung und Testung von Kompositen mit den Glasfüllstoffen

Durch Mischen folgender Komponenten wurde eine Harzmatrix hergestellt:

| **Menge** | **Komponente** |
|---|---|
| 15,0 Gew.-% | 10-Methacryloyloxydecyldihydrogenphosphat (MDP) (Adhäsivmonomer) |
| 3,0 Gew.-% | 2-Methacryloyloxyethyldihydrogenphosphat (Adhäsivmonomer) |
| 66,0 Gew.-% | Urethandimethacrylat (UDMA) (Vernetzermonomer), |
| 15,0 Gew.-% | 2-(Methacryloyloxy)ethylacetoacetat (Verdünnermonomer) |
| 0,3 Gew.-% | Campherchinon (Initiator), |
| 0,6 Gew.-% | 4-(Dimethylamino)-benzoesäureethylester (Initiator) |
| 0,1 Gew.-% | Additive (Stabilisatoren und UV-Absorber) |

Mit dieser Harzmatrix und den Glasfüllern aus Bezugsbeispiel 1 bzw. Beispiel 2 wurden jeweils Kompositpasten folgender Zusammensetzung hergestellt:

| **Komponente** | **Vergleichs-beispiel 3** | **Beispiel 4** |
|---|---|---|
| Harzmatrix | 22,8 Gew.-% | 22,8 Gew.-% |
| Glasfüller aus | 37,1 Gew.-% | - |
| Bezugsbeispiel 1 Glasfüller aus | - | 37,1 Ges.-% |
| Beispiel 2 Isofüller* | 37, 9 Gew.-% | 37, 9 Gew.-% |
| Bentone®-Paste** | 2,2 Gew.-% | 2,2 Ges.-% |

| | | |
|---|---|---|
| * Isofüller ist ein heißgehärteter Kompositfüller, der auf die gewünschte Korngröße vermahlen wurde und hergestellt wurde aus: Bis-GMA (8,80 Gew.-%), UDMA (6,60 Gew.-%), 1,10-Decandioldimethacrylat (5,93 Gew.-%), Dibenzoylperoxid + 2,6-Di-tert.-butyl-4-methylphenol (zusammen 0,67 Gew.-%), Glasfüller, mittlere Korngröße 0,4 µm (53,0 Gew.-%) und YbF₃ (25,0 Gew.-%) ** Bentone® 38 (Fa. Rheox Inc., Hightown, USA) ist ein organisch modifiziertes Mg-Schichtsilicat (Hectorit) und wurde als 12,5-%ige Dispersion in die Harzmatrix eingebracht. | | |

Dazu wurde die Monomermischung vorgelegt, dann die Bentone®-Paste und die erste Hälfte der Glasfüllermenge zugegeben und die Masse homogen verknetet. Danach wurde die zweite Portion der Glasfüllermenge zugegeben und verknetet. Anschließend wurde die erste Hälfte Isofüller zugegeben und verknetet, und schließlich der Rest an Isofüller zugegeben und die Paste homogen verknetet. Zuletzt wurde die Paste 15 min bei 20.000 Pa entlüftet.

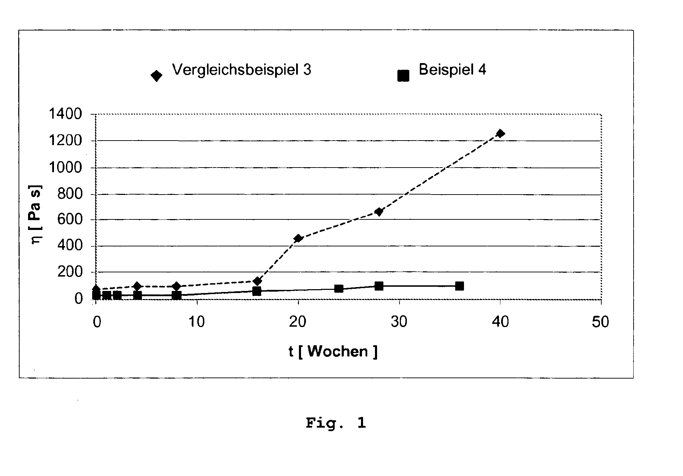

Die Viskosität der Kompositpasten wurde in Abhängigkeit von der Lagerungszeit der Pasten bei 50°C mit Hilfe eines Bohlin CVO-10 Rheometers (Fa. Bohlin, Pforzheim) mit einer Schergeschwindigkeit von 1 s⁻¹ bei 23°C gemessen. Der Einfluss der Glasfüllerbehandlung auf die Viskosität η in Abhängigkeit der Zeit t ist in Fig. 1 dargestellt.

Die Ergebnisse belegen eine deutliche Verbesserung der Lagerstabilität der erfindungsgemäßen Kompositpaste.

Aus den Kompositpasten wurden nach der EN ISO 6872: 1998 in Stahlformen, die mit einer 3%igen V-Wachslösung in Hexan isoliert wurden, Prüfkörperscheiben (Höhe: 1,2 mm, Durchmesser: 15 mm) bei einer Bestrahlungszeit von 2 mal 3 min beidseitig mit einem Spectramat® (Fa. Ivoclar Vivadent AG, Liechtenstein) hergestellt und die Biaxialfestigkeit nach 24 h Wasserlagerung der Prüfkörper bei 37°C gemessen:

| | |
|---|---|
| Vergleichsbeispiel 3: | 115,6 ± 9,4 MPa, |
| Beispiel 4: | 117,1 ± 14,7 MPa. |

Der Vergleich der beiden Komposite zeigt, dass die PVC-Beschichtung zu keiner nachteiligen Beeinflussung der mechanischen Eigenschaften führt.

## Patentansprüche

1. Füllstoff für Dentalwerkstoffe auf der Basis von Glaspartikeln, **dadurch gekennzeichnet, dass** die Oberfläche der Glaspartikel mindestens teilweise mit einem Homo- oder Copolymer von Vinylchlorid beschichtet ist.

2. Füllstoff nach Anspruch 1, bei dem das Homo- oder Copolymer von Vinylchlorid ein zahlenmittleres Molekulargewicht Mₙ im Bereich von 30.000 bis 130.000 aufweist.

3. Füllstoff nach einem der vorhergehenden Ansprüche, bei dem das Glas ein Silicatglas ist.

4. Füllstoff nach Anspruch 3, bei dem das Glas Bariumoxid und/oder Strontiumoxid enthält.

5. Füllstoff nach einem der vorhergehenden Ansprüche, bei dem das Glas 15 bis 35 Gew.-% Bariumoxid und/oder 15 bis 25 Gew.-% Strontiumoxid enthält.

6. Füllstoff nach einem der vorhergehenden Ansprüche, bei dem die Glaspartikel aus einem Glas bestehen, das in mikronisierter Form bei Dispersion in Wasser eine alkalische Reaktion zeigt.

7. Füllstoff nach einem der vorhergehenden Ansprüche, bei dem das Glas einen Fluoridgehalt von < 1 Gew.-% aufweist.

8. Füllstoff nach einem der vorhergehenden Ansprüche, bei dem das Glas röntgenopak ist.

9. Füllstoff nach einem der vorhergehenden Ansprüche, bei dem das Glas Barium-Silicat-Glas oder Strontium-Silicat-Glas ist.

10. Füllstoff nach Anspruch 9, bei dem das Glas 40 bis 60 Ges.-% SiO₂, 10 bis 15 Gew.-% Al₂O₃, 10 bis 20 Ges.-% B₂O₃ und 15 bis 35 Gew.-% BaO enthält.

11. Füllstoff nach Anspruch 9, bei dem das Glas 40 bis 60 Ges.-% SiO₂, 10 bis 15 Gew.-% Al₂O₃, 10 bis 20 Ges.-% B₂O₃, 0 bis 3 Gew.-% BaO und 15 bis 25 Gew.-% SrO enthält.

12. Füllstoff nach einem der vorhergehenden Ansprüche, bei dem das Glas silanisiert ist.

13. Füllstoff nach einem der vorhergehenden Ansprüche, bei dem die Glaspartikel auf der Oberfläche Polyvinylchlorid oder ein Copolymer von Vinylchlorid aufweisen, das mindestens 80 mol-% polymerisierte Vinylchlorid-Einheiten und bis zu 20 mol % polymerisierte Einheiten eines oder mehrerer Comonomere enthält.

14. Füllstoff nach einem der vorhergehenden Ansprüche, bei dem die Glaspartikel ohne das Homo- oder Copolymer von Vinylchlorid eine mittlere Partikelgröße im Bereich von 0,01 bis 10 µm aufweisen.

15. Füllstoff nach einem der vorhergehenden Ansprüche, bei dem die Glaspartikel eine spezifische Oberfläche - bestimmt mit der BET-Methode nach ISO 9277:1995 - von 1 bis 30 m²/g aufweisen.

16. Verfahren zur Herstellung eines Füllstoff für Dentalwerkstoffe auf der Basis von Glaspartikeln, bei dem man Glaspartikel in einer Lösung eines Homo- oder Copolymers von Vinylchlorid in einem organischen Lösungsmittel dispergiert, das Lösungsmittel anschließend entfernt und dann den Füllstoff bei einer Temperatur, die mindestens ca. 30 °C unterhalb der Glasübergangstemperatur des Homo- oder Copolymers von Vinylchlorid liegt, trocknet.

17. Verfahren nach Anspruch 16, bei dem das organische Lösungsmittel ausgewählt ist aus cyclischen Ketonen, Dimethylformamid, Dimethylsulfoxid, Tetrahydrofuran und Mischungen derselben.

18. Verfahren nach Anspruch 16 oder 17, bei dem das Glas, die Glaspartikel und/oder das Copolymer von Vinylchlorid wie in einem der Ansprüche 1 bis 15 definiert sind.

19. Füllstoff nach Anspruch 1, **dadurch gekennzeichnet, dass** er gemäß einem Verfahren nach einem der Ansprüche 16 bis 18 erhältlich ist.

20. Deritalwerkstoff, der
(a) 1 bis 85 Gew.-% Füllstoff nach einem der Ansprüche 1 bis 15 oder 19,
(b) 0,1 bis 5,0 Gew.-% Initiator für die radikalische Polymerisation und
(c) 1 bis 70 Gew.-% eines oder mehrerer säuregruppenfreier radikalisch polymerisierbarer Monomere und
(d) 1 bis 30 Gew.-% eines oder mehrerer säuregruppenhaltiger radikalisch polymerisierbarer Monomere enthält, jeweils bezogen auf das Gesamtgewicht des Dentalwerkstoffs.

21. Verwendung eines Füllstoffs nach einem der Ansprüche 1 bis 15 oder 19 zur Herstellung eines selbsthaftenden Komposits.

22. Verwendung eines Füllstoffs nach einem der Ansprüche 1 bis 15 oder 19 zur Verbesserung der Lagerstabilität eines Dentalwerkstoffs.

## Claims

1. A filler for dental materials based on glass particles, **characterised in that** the surface of the glass particle is at least partly coated with a homopolymer or copolymer of vinyl chloride.

2. The filler according to claim 1, in which the number average molecular weight Mₙ of the homopolymer or copolymer of vinyl chloride is from 30 000 to 130 000.

3. The filler according to one of the previous claims, in which the glass is a silicate glass.

4. The filler according to claim 3, in which the glass comprises barium oxide and/or strontium oxide.

5. The filler according to one of the previous claims, in which the glass comprises 15 to 35 wt % barium oxide and/or 15 to 25 wt % strontium oxide.

6. The filler according to one of the previous claims, in which the glass particles consist of a glass that in micronised form exhibits an alkaline reaction on dispersion in water.

7. The filler according to one of the previous claims, in which the glass has a fluoride content of < 1 wt %.

8. The filler according to one of the previous claims, in which the glass is X-ray opaque.

9. The filler according to one of the previous claims, in which the glass is barium silicate glass or strontium silicate glass.

10. The filler according to claim 9, in which the glass comprises 40 to 60 wt % SiO₂, 10 to 15 wt % Al₂O₃, 10 to 20 wt % B₂O₃ and 15 to 35 wt % BaO.

11. The filler according to claim 9, in which the glass comprises 40 to 60 wt % SiO₂, 10 to 15 wt % Al₂O₃, 10 to 20 wt % B₂O₃, 0 to 3 wt % BaO and 15 to 25 wt % SrO.

12. The filler according to one of the previous claims, in which the glass is silanised.

13. The filler according to one of the previous claims, in which poly(vinyl chloride) or a copolymer of vinyl chloride is present on the surfaces of the glass particles, wherein said copolymer comprises at least 80 mol % of polymerised vinyl chloride units and up to 20 mol % of polymerised units of one or more comonomers.

14. The filler according to one of the previous claims, in which the mean particle size of the glass particles without the homopolymer or copolymer of vinyl chloride is between 0.01 to 10 µm.

15. The filler according to one of the previous claims, in which the glass particles have a specific surface area - determined by the BET method according to ISO 9277:1995 - of 1 to 30 m²/g.

16. A process for the preparation of a glass particle-based filler for dental materials by dispersing glass particles in a solution of a homopolymer or copolymer of vinyl chloride in an organic solvent, subsequently removing the solvent and then drying the filler at a temperature that is at least 30 °C below the glass transition temperature of the homopolymer or copolymer of vinyl chloride.

17. The process according to claim 16, in which the organic solvent is selected from cyclic ketones, dimethyl formamide, dimethyl sulfoxide, tetrahydrofuran and mixtures thereof.

18. The process according to claim 16 or 17, in which the glass, the glass particles and/or the copolymer of vinyl chloride are as defined in one of claims 1 to 15.

19. The filler according to claim 1, **characterised in that** it is obtainable by a process according to one of claims 16 to 18.

20. A dental material, comprising
a) 1 to 85 wt % filler according to one of claims 1 to 15 or 19,
b) 0.1 to 5.0 wt % initiator for the radical polymerisation and
c) 1 to 70 wt % of one or more acid group-free radically polymerisable monomers and
d) 1 to 30 wt % of one or more acid group-containing radically polymerisable monomers,
each based on the total weight of the dental material.

21. Use of a filler according to one of claims 1 to 15 or 19 for manufacturing a self-adhesive composite.

22. Use of a filler according to one of claims 1 to 15 or 19 for improving the storage stability of a dental material.

## Revendications

1. Charge pour matériaux dentaires, à base de particules de verre, **caractérisée en ce que** la surface des particules de verre est au moins partiellement revêtue avec un homopolymère ou copolymère de chlorure de vinyle.

2. Charge selon la revendication 1, dans laquelle l'homopolymère ou le copolymère de chlorure de vinyle présente une masse moléculaire moyenne en nombre Mₙ dans la plage de 30 000 à 130 000.

3. Charge selon l'une quelconque des revendications précédentes, dans laquelle le verre est un verre de silicate.

4. Charge selon la revendication 3, dans laquelle le verre contient de l'oxyde de baryum et/ou de l'oxyde de strontium.

5. Charge selon l'une quelconque des revendications précédentes, dans laquelle le verre contient de 15 à 35 % en poids d'oxyde de baryum et/ou de 15 à 25 % en poids d'oxyde de strontium.

6. Charge selon l'une quelconque des revendications précédentes, dans laquelle les particules de verre consistent en un verre qui, sous forme micronisée lors de dispersion dans de l'eau, présente une réaction alcaline.

7. Charge selon l'une quelconque des revendications précédentes, dans laquelle le verre présente une teneur en fluorure de < 1 % en poids.

8. Charge selon l'une quelconque des revendications précédentes, dans laquelle le verre est opaque aux rayons X.

9. Charge selon l'une quelconque des revendications précédentes, dans laquelle le verre est un verre de silicate-baryum ou de silicate-strontium.

10. Charge selon la revendication 9, dans laquelle le verre contient de 40 à 60 % en poids de SiO₂, de 10 à 15 % en poids d'Al₂O₃, de 10 à 20 % en poids de B₂O₃ et de 15 à 35 % en poids de BaO.

11. Charge selon la revendication 9, dans laquelle le verre contient de 40 à 60 % en poids de SiO₂, de 10 à 15 % en poids d'Al₂O₃, de 10 à 20 % en poids de B₂O₃, de 0 à 3 % en poids de BaO et de 15 à 25 % en poids de SrO.

12. Charge selon l'une quelconque des revendications précédentes, dans laquelle le verre est silané.

13. Charge selon l'une quelconque des revendications précédentes, dans laquelle les particules de verre comportent à la surface du poly(chlorure de vinyle) ou un copolymère de chlorure de vinyle qui contient au moins 80 % en moles de motifs chlorure de vinyle polymérisés et jusqu'à 20 % en moles de motifs polymérisés d'un ou plusieurs comonomères.

14. Charge selon l'une quelconque des revendications précédentes, dans laquelle les particules de verre ont, sans l'homopolymère ou le copolymère de chlorure de vinyle, une taille moyenne de particule dans la plage de 0,01 à 10 µm.

15. Charge selon l'une quelconque des revendications précédentes, dans laquelle les particules de verre ont une surface spécifique - déterminée par la méthode BET selon ISO 9277:1995 - de 1 à 30 m²/g.

16. Procédé pour la production d'une charge pour matériaux dentaires à base de particules de verre, dans lequel on disperse des particules de verre dans une solution d'un homopolymère ou copolymère de chlorure de vinyle dans un solvant organique, puis on élimine le solvant et ensuite on sèche la charge à une température qui est inférieure d'au moins environ 30 °C à la température de transition vitreuse de l'homopolymère ou du copolymère de chlorure de vinyle.

17. Procédé selon la revendication 16, dans lequel le solvant organique est choisi parmi des cétones cycliques, le diméthylformamide, le diméthylsulfoxyde, le tétrahydrofurane et des mélanges de ceux-ci.

18. Procédé selon la revendication 16 ou 17, dans lequel le verre, les particules de verre et/ou le copolymère de chlorure de vinyle sont tels que définis dans l'une quelconque des revendications 1 à 15.

19. Charge selon la revendication 1, **caractérisée en ce qu'**elle peut être obtenue conformément à un procédé selon l'une quelconque des revendications 16 à 18.

20. Matériau dentaire, qui contient
(a) 1 à 85 % en poids de charge selon l'une quelconque des revendications 1 à 15 ou 19,
(b) 0,1 à 5,0 % en poids d'amorceur pour la polymérisation radicalaire et
(c) 1 à 70 % en poids d'un ou plusieurs monomètres polymérisables par voie radicalaire, exempts de groupes acides, et
(d) 1 à 30 % en poids d'un ou plusieurs monomères polymérisables par voie radicalaire, contenant des groupes acides, ,
chaque fois par rapport au poids total du matériau dentaire.

21. Utilisation d'une charge selon l'une quelconque des revendications 1 à 15 ou 19, pour la production d'un composite autoadhésif.

22. Utilisation d'une charge selon l'une quelconque des revendications 1 à 15 ou 19, pour l'amélioration de la stabilité au stockage d'un matériau dentaire.
